# EUROPEAN PATENT SPECIFICATION

(11) **EP 4 247 275 B1**
(45) Date of publication and mention of the grant of the patent: **04.11.2026**
(21) Application number: 21819679.8
(22) Date of filing: 11.11.2021
(51) Int. Cl.: A61B 17/122, A61B 17/128, A61B 17/34

(54) **SURGICAL CLIP PACKS COUPLEABLE TO SURGICAL ACCESS DEVICES**
AN CHIRURGISCHE ZUGANGSVORRICHTUNGEN KOPPELBARE CHIRURGISCHE KLAMMERPACKUNGEN
ENSEMBLES PINCES CHIRURGICALES POUVANT ÊTRE COUPLÉS À DES DISPOSITIFS D'ACCÈS CHIRURGICAUX

(30) Priority: 20.11.2020 US 202063116362 P; 09.11.2021 US 202117522072
(43) Date of publication of application: 27.09.2023
(73) Proprietor: Covidien LP, Mansfield, MA 02048 (US)
(72) Inventor: BARIL, Jacob C., Norwalk, Connecticut 06851 (US); EBERSOLE, Garrett P., Hamden, Connecticut 06517 (US); BANERJEE, Saumya, Collinsville, Connecticut 06019 (US); THOMAS, Justin J., New Haven, Connecticut 06512 (US); PILLETERE, Roy J., Middletown, Connecticut 06457 (US); DININO, Matthew A., Newington, Connecticut 06111 (US); ROY, Nicolette L., Windsor Locks, Connecticut 06096 (US)
(74) Representative: Gray, James
(86) International application number: PCT/US2021/058892
(87) International publication number: WO 2022/108812

(56) References cited:
- WO-A2-2019/089300
- US-B1- 6 419 682
- "Schnappverbindungen aus Kunststoff", 1 January 1989, EXPERT VERLAG, Ehningen bei Böblingen, ISBN: 978-3-8169-0488-5, article UWE DELPY ET AL: "Schnappverbindungen aus Kunststoff", pages: 1,2,9,36, XP055428286

## Description

### CROSS-REFERENCE TO RELATED APPLICATION

The present application claims the benefit of and priority to U.S. Provisional Application No. 63/116,362, filed on November 20, 2020.

### FIELD

The present disclosure is generally related to surgical devices. More particularly, this disclosure relates to a surgical clip pack configured to couple to a surgical access device.

### BACKGROUND

Many surgical procedures require vessels, veins, or other tissues of the body to be tied, or ligated, during the procedure. Specialized, non-absorbable ligation clips, typically made of a polymer, have been developed for this purpose. The clips are often applied using a single-fire clip applier that applies a clip over vessels laparoscopically. A surgeon needs to remove the single fire clip applier from the surgical working space via a surgical access device, such as a cannula, in order to reload the clip applier with a new ligation clip.

The clips are generally packaged together in small "refill" packs with a couple of clips disposed of therein. The refill packs and the clips may be easily dropped outside the sterile field or may otherwise be lost or misplaced due to their small size and requiring the clinician to obtain a new refill pack. Each dropped or lost clip or refill pack adds to the overall cost of the surgery as each one needs to be replaced. Furthermore, surgeons or medical staff may spend extra time looking for the dropped or misplaced refill packs or clips, unnecessarily extending a surgery. If the surgeon is unfamiliar with the setup in the room, they may not always remember or know where the refill pack is kept causing a small delay looking for the refill pack of clips. Accordingly, a device that keeps the refill packs and therefore the clips within the surgical sterile field that is easily accessible to the surgeon or medical staff in a similar location for any procedure regardless of the surgery or room setup is desirable. WO 2019/089300 A2 (Ethicon LLC) discloses a clip applier that engages with a clip magazine housing surgical clips for supplying the clip applier, both the magazine and the applier provided at a distal end of the access device.

### SUMMARY

The invention is defined in the attached independent claims to which reference should now be made. Further, optional features may be found in the sub-claims appended thereto. This disclosure generally relates to a surgical clip pack. The surgical clip pack includes an annular frame configured to engage a portion of a surgical access device. The frame includes a storage compartment and an annulus defining a lumen that has a diameter at least as large as a diameter of an opening of the surgical access device. A plurality of receptacles are disposed within the storage compartment, each receptacle configured to house a surgical fastener.

In aspects, the frame may include at least one tab configured to engage a slot disposed on a proximal portion of the surgical access device.

The storage region is disposed circumferentially about the frame. Each receptacle may be configured to house at least one surgical fastener.

In yet another aspect, the surgical fastener may be a surgical clip.

In another aspect, at least one receptacle of the storage region may be configured to house a surgical fastener of a different size than another surgical fastener housed by at least one other receptacle.

In aspects, the storage compartment may surround about a third of the annulus.

In additional aspects, at least five receptacles may be disposed within the storage compartment.

In disclosed aspects, the receptacle may include a pin disposed within a proximal portion thereof. The pin may be configured to fit within an elbow of a surgical fastener.

In further aspects, the frame may include two tabs. The tabs may include a shelf at distal portions thereof. The shelf may be configured to cause the tabs to flex radially inwards when inserted into a slot of the surgical access device until the shelf exits the slot. The shelf may extend over an edge of the slot thereby securing the surgical clip pack to the surgical access device.

In accordance with another aspect of this disclosure, a surgical clip pack includes a ring configured to couple to a proximal portion of a surgical access device. The surgical clip pack also includes an arm coupled to a side of the ring. The arm includes a plurality of receptacles. Each receptacle is configured to house at least one surgical fastener.

In a further aspect, the ring may include tabs configured to engage a slot disposed proximally on the surgical access device.

In further aspects, the ring may include two tabs. The tabs may include a shelf at distal portions thereof. The shelf may be configured to cause the tabs to flex radially inwards when inserted into a slot of the surgical access device until the shelf exits the slot. The shelf may extend over an edge of the slot thereby securing the surgical clip pack to the surgical access device.

In another aspect, the ring may define an aperture that has a diameter that is at least as large as a diameter of a proximal opening of the surgical access device.

In disclosed aspects, the arm may extend circumferentially about at least a portion of the ring.

In additional aspects, the surgical fastener may include bars at distal ends of the arms of the surgical fasteners. The bars may be configured to press against an internal wall of the receptacle to hold the surgical fastener in the receptacle.

In aspects, the surgical fastener may be a surgical clip.

In another aspect, at least one receptacle of the storage region may be configured to house a surgical fastener of a different size than another surgical fastener housed by at least one other receptacle.

In an aspect, each receptacle of the plurality of receptacles may include a pin configured to stabilize the surgical fastener therein.

In yet further aspects, the pin may be configured to fit into an elbow of the surgical fastener such that the pin holds the surgical fastener in the receptacle.

The details of one or more aspects of the disclosure are set forth in the accompanying drawings and the description below. Other features, objects, and advantages of the techniques described in this disclosure will be apparent from the description and drawings, and from the claims.

### DESCRIPTION OF DRAWINGS

The accompanying drawings, which are incorporated in and constitute a part of this specification, illustrate aspects and features of the disclosure and, together with the detailed description below, serve to further explain the disclosure, in which:
FIG. 1 is a perspective view of a surgical clip pack coupled to a surgical access device;
FIG. 2 is an exploded perspective view of the surgical clip pack separated from the surgical access device shown in FIG. 1;
FIG. 3 is a top view of the surgical clip pack of FIGS. 1 and 2;
FIG. 4 is a side cross-sectional view of the surgical clip pack coupled to the surgical access device taken along section line 4-4 of FIG. 1;
FIG. 5 is a perspective view of a surgical clip applier inserted through the surgical clip pack and surgical access device of FIG. 1;
FIG. 6 is a perspective view of the surgical clip applier removed from the surgical clip pack and surgical access device of FIG. 1;
FIG. 7 is a perspective view of a surgical clip applier being reloaded with a clip stored in the surgical clip pack of FIG. 1;
FIG. 8 is a side cross-sectional view taken along section line 8-8 of FIG. 6;
FIG. 9 is a side cross-sectional view taken along section line 9-9 of FIG. 7; and
FIG. 10 is a perspective view of the reloaded surgical clip applier removed from a receptacle of the surgical clip pack.

Further details and various aspects of this disclosure are described in more detail below with reference to the appended figures.

### DETAILED DESCRIPTION

Aspects of the presently disclosed surgical clip pack are described in detail with reference to the drawings, in which like reference numerals designate identical or corresponding elements in each of the several views. However, it is to be understood that the disclosed devices are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

Descriptions of technical features of an illustrative surgical clip pack in accordance with the disclosure should typically be considered as available and applicable to other similar features of another device of the disclosure. Accordingly, technical features described herein in connection with one illustrative surgical clip pack may apply to other devices of the disclosure, and thus duplicative descriptions may be omitted herein.

As used herein the term "distal" refers to that portion of a surgical access device, or component thereof, farther from the user, while the term "proximal" refers to that portion of the surgical access device, or component thereof, closer to the user.

This disclosure relates to a surgical clip pack for storing and providing surgical clips, such as ligation clips. The surgical clip pack is configured to attach to a surgical access device, such as a trocar or cannula such that surgical clips stored in the surgical clip pack are easily accessible. During a surgical procedure, a surgeon can quickly and efficiently reload a single fire surgical clip applier with a new clip from the surgical clip pack. Since the surgical clip pack is coupled to the surgical access device, time is saved that may have otherwise been spent looking for a traditional clip pack having additional surgical clips to reload the surgical clip applier. Further, since the clip pack of this disclosure is coupled to the surgical access device, the opportunity for the additional surgical clips to become contaminated is reduced. While the surgical clip pack is discussed herein with reference to surgical clips, other small devices or instruments used for surgical procedures, such as sutures, needles, or staples may also be stored in and provided by the surgical clip pack.

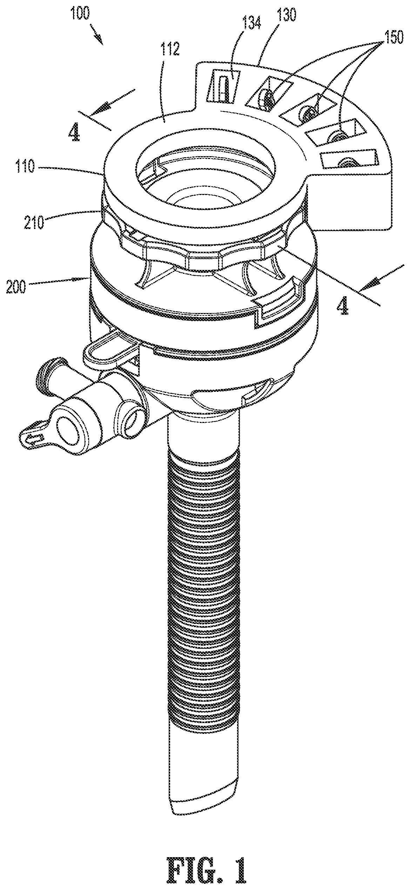

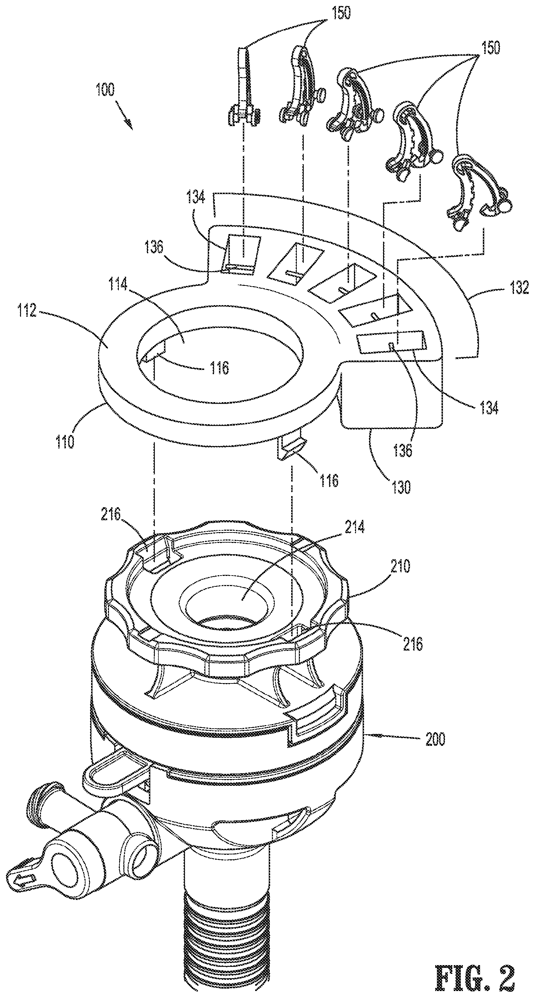

With reference to FIGS. 1 and 2, a surgical clip pack 100 for storing and providing surgical fasteners, such as surgical clips 150, generally includes a frame 110 and a storage compartment 130 extending outwardly from the frame 110. The frame 110 may be an annulus 112 defining a lumen 114. The storage compartment 130 includes a storage region 132 with receptacles 134. In aspects, the storage compartment 130 includes just one receptacle 134. In aspects, storage compartment 130 is an arm extending from the frame 110 and includes at least one receptacle 134. The surgical clip pack 100 provides easy and convenient access to the surgical clips 150 during a surgical procedure.

The surgical clip pack 100 is configured to engage a proximal portion of the surgical access device 200, such as housing 210. The frame 110 includes tabs 116 configured to removably couple to slots 216 of the housing 210. The tabs 116 are configured to snap into the slots 216 such that the surgical clip pack 100 is secured to the surgical access device 200.

The annulus 112 of the frame 110 includes a lumen 114 having a diameter that is at least as large as a diameter of an opening 214 of the housing 210 of the surgical access device 200. The frame 110 is configured such that a surgical instrument may be inserted through the lumen 114 without interference from the surgical clip pack 100.

In aspects, the frame 110 may be "C" shaped and configured to snap onto a tube or cylindrical portion of a surgical access device 200.

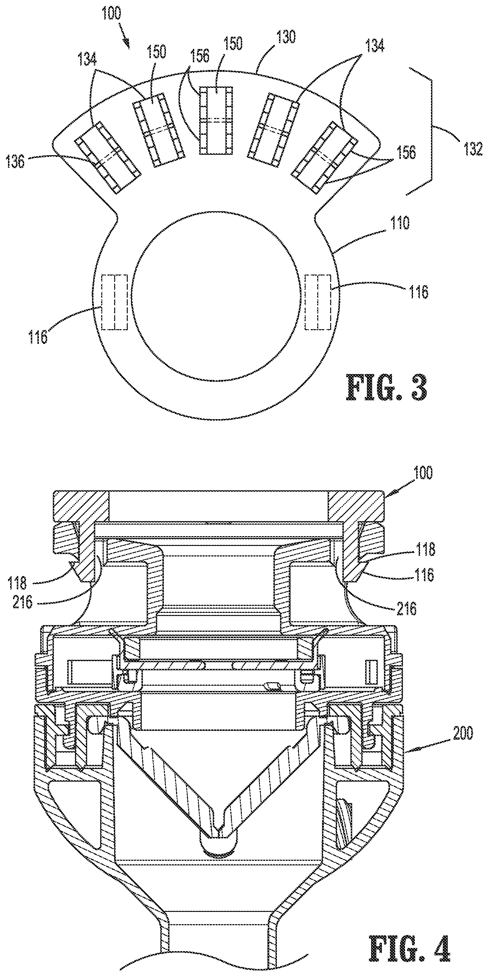

Referring to FIG. 3, the storage compartment 130 extends from frame 110 and is configured to surround a portion of frame 110. For example, storage compartment 130 surrounds about a third of the frame 110 as illustrated. In aspects, the storage compartment 130 may completely surround the frame 110. The storage region 132 of the storage compartment 130 is arranged about the storage compartment 130 and circumferentially about the frame 110. Each receptacle 134 of the storage region 132 is configured to house a surgical clip 150 (FIG. 2).

In aspects, the storage region 132 includes five receptacles 134 arranged within the storage compartment 130. Each receptacle 134 of the storage region 132 may be configured to house a surgical clip 150 of the same size or surgical clips 150 of varying sizes.

For example, three medium-sized surgical clips 150 may be stored in three separate receptacles 134, one large-sized surgical clip 150 may be stored in another receptacle 134, and a small-sized surgical clip 150 may be stored in a remaining receptacle 134. The number of surgical clips 150 that may be provided by the surgical clip pack 100 may vary depending on the number of receptacles 134 of the storage region 132.

In aspects, sutures, needles, or any other small surgical device may be stored in the storage region 132.

Alternatively, storage compartment 130 may include a single receptacle for housing a traditional packaged clip pack (not shown). For example, a container having clips disposed thereon may be placed into the receptacle 132 of the storage compartment 130 and held in place via a retention mechanism, such as a spring arm.

In yet another aspect, the storage compartment is a cartridge extending from the frame 110 configured to hold surgical fasteners.

With reference to FIG. 4, a side cross-sectional view of the surgical clip pack 100 taken along line 4-4 of FIG.1 is shown. The tabs 116 extending from the frame 110 are configured to couple the surgical clip pack 100 to a housing 210 of the surgical access device 200 via slots 216 of the housing 210. The tabs 116 include a shelf 118, such that, when the tabs 116 are inserted into slot 216, the tabs 116 are flexed radially inwards until the shelf 118 is inserted past an end of slot 216. The tabs 116 then return to an approximately unflexed position, securing the surgical clip pack 100 to the surgical access device 200. To decouple the surgical clip pack 100 from the housing 210 of the surgical access device 200, the tabs 116 are pressed inwards and pushed up such that shelf 118 may pass through slot 216.

In aspects, any tabs or snaps known by those of ordinary skill in the art may be used to couple and secure the surgical clip pack 100 to the surgical access device 200. For example, instead of shelf 118, the tabs 116 may include rounded protrusions at distal ends thereof that snap into rounded cavities of the slots 216 to secure the surgical clip pack 100 to the surgical access device 200 and may be removed with sufficient force pulling on the surgical clip pack 100.

In aspects, the surgical clip pack 100 may include a wrap or seal (not shown) that is removed after coupling the surgical clip pack 100 to the surgical access device 200. In aspects, the wrap or seal covers only a top face of the storage region 132 and receptacles 134. The wrap or seal may be configured to prevent contamination of the surgical clips 150 stored in the surgical clip pack 100. The wrap or seal may be removed in a sterile environment just prior to use.

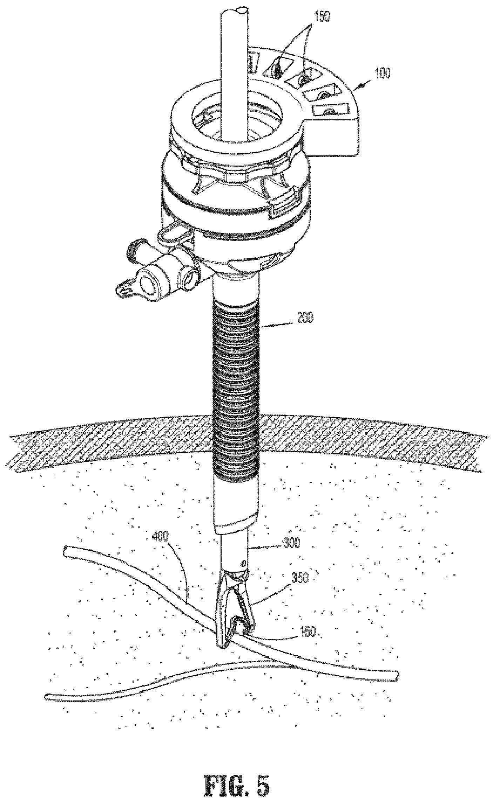

With reference to FIG. 5, prior to inserting a surgical clip applier 300 into the surgical access device 200, the surgical clip pack 100 is coupled to the surgical access device 200 via the tabs 116 so a surgeon or medical professional has easy access to additional surgical clips 150 or other surgical devices and fasteners. The surgeon may then insert a surgical instrument, such as a surgical clip applier 300, through the lumen 114 of surgical clip pack 100 and through the opening 214 into a lumen of the surgical access device 200. The surgeon or medical professional may clamp or otherwise restrict vessels, veins, arteries, or other tissues using a surgical clip 150 via the surgical clip applier 300. A surgical clip 150 is applied to a vascular structure 400 using the surgical clip applier 300.

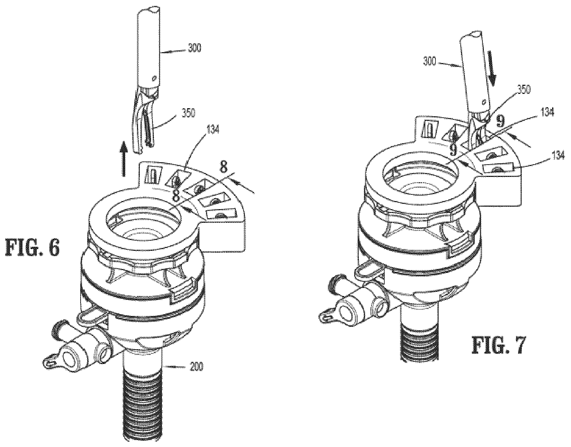

With reference to FIGS. 6 and 7, the surgeon or medical professional then removes the surgical clip applier 300 from the surgical access device 200 and surgical clip pack 100, so that the surgical clip applier 300 may be reloaded with a new surgical clip 150. Since the surgical clip pack 100 is near the operation site, the surgical clip applier 300 is easily reloaded with additional surgical clips 150 while cutting down on time spent looking for additional clips in the operating room. The surgical clip applier 300 is reloaded by inserting the surgical clip applier 300 into a receptacle 134 to grasp a surgical clip 150 stored therein. Since the surgical clip applier 300 may be reloaded by inserting a distal end of the surgical clip applier 300 into a receptacle 134 having a surgical fastener 150 stored therein, the chances a surgical clip 150 is dropped and thereby contaminated is reduced.

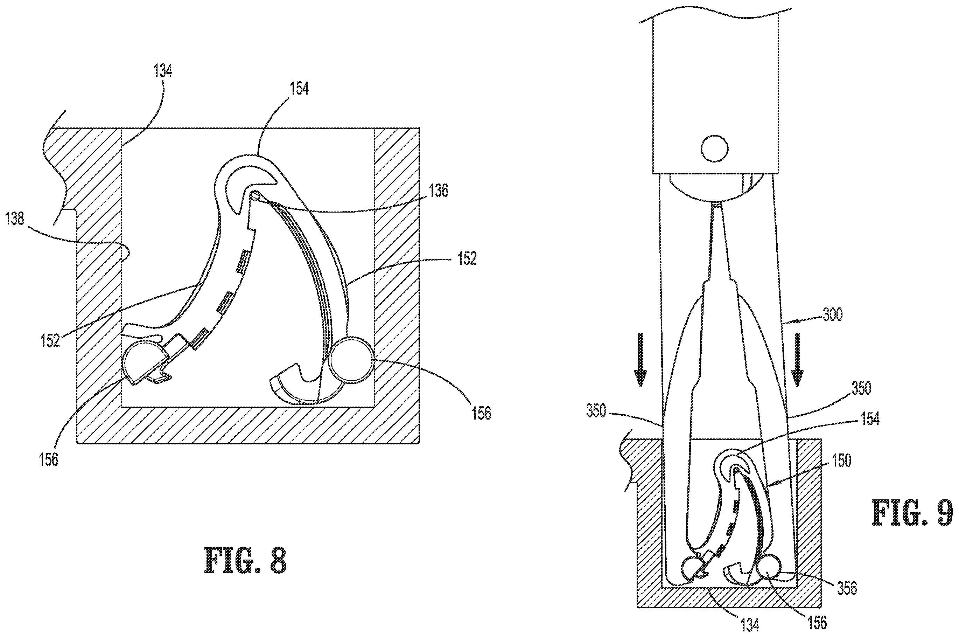

With reference to FIGS. 8 and 9, side views of the receptacles 134 taken along lines 8-8 and 9-9 of FIGS. 6 and 7, respectively, are shown. Each receptacle 134 may include a pin 136 disposed near an upper portion of the receptacle 134 and configured to hold a surgical clip 150. Each arm 152 of a surgical clip 150 would be positioned on either side of the pin 136, and the pin 136 tucked into an elbow 154 of the surgical clip 150. Thus, the pin 136 helps stabilize the surgical clip 150 within the receptacle 134. In aspects, the pin 136 holds the surgical clip 150 within the receptacle 134 via a friction fit with the elbow 154 of the surgical clip.

The arms 152 of the surgical clip 150 are slightly depressed within the receptacle 134. The arms 152 may press outward against the internal walls 138 of the receptacle 134 with sufficient force to hold the surgical clip 150 in the receptacle 134 (i.e., friction fit). The distal ends of the arms 152 may include bars 156 configured to fit with notches 356 of jaws 350 of the surgical clip applier 300. In aspects, the distal ends of the surgical clip 150 or the bars 156, may be pressed against the internal walls 138 of the receptacle 134 with sufficient force to hold the surgical clip 150 in receptacle 134.

As the surgeon or medical professional inserts the clip applier 300 into the receptacle 134 to reload the surgical clip applier 300 with the new surgical clip 150, the jaws 350 of the surgical clip applier 300 further depress the surgical clip arms 152 towards one another. The jaws 350 are then in a partially closed configuration as illustrated. The surgeon continues to insert the surgical clip applier 300 into the receptacle 134 until the notches 356 engage the bars 156, thus securing the surgical clip 150 in the jaws 350.

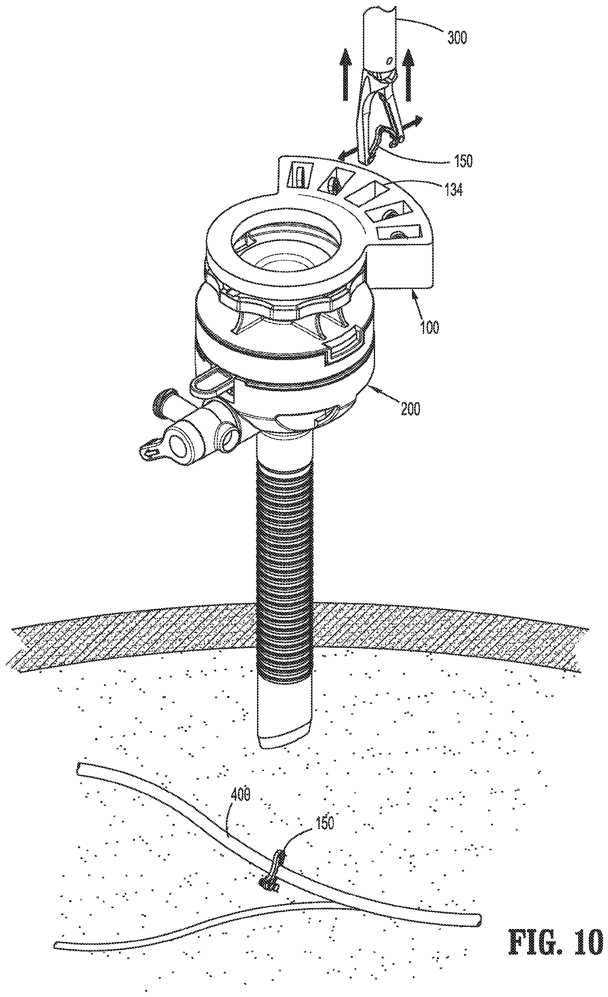

With reference to FIG. 10, after the surgical clip 150 is secured by the jaws 350, the surgeon may remove the surgical clip applier 300. The jaws 350 may be extended into an open position, thus pulling the arms 152 away from each such that they are no longer depressed. The surgeon then repeats the process by inserting the surgical clip applier 300 into the surgical clip pack 100 and surgical access device 200 to apply the surgical clip 150 as needed.

Persons skilled in the art will understand that the devices specifically described herein and illustrated in the accompanying drawings are non-limiting exemplary aspects of the disclosure. It is envisioned that the elements and features illustrated or described in connection with one exemplary aspect may be combined with the elements and features of another without departing from the scope of the disclosure. As well, one skilled in the art will appreciate further features and advantages of the disclosure based on the above-described aspects. Accordingly, the disclosure is not to be limited by what has been particularly shown and described, except as indicated by the appended claims.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

## Claims

1. A surgical clip pack (100) comprising:
an annular frame (110) configured to engage a proximal portion of a surgical access device (200), the frame including:
a storage compartment (130); and
an annulus (112) defining a lumen (114) that has a diameter at least as large as a diameter of an opening of the surgical access device; and
a plurality of receptacles (134) disposed within the storage compartment and disposed circumferentially about the frame, each receptacle of the plurality of receptacles configured to house at least one surgical fastener (150).

2. The surgical clip pack of claim 1, wherein the frame (110) includes at least one tab (116) configured to engage a slot (216) disposed on the proximal portion of the surgical access device (200).

3. The surgical clip pack of any preceding claim, wherein at least one receptacle (134) of the plurality of receptacles is configured to house a surgical fastener (150) of a different size than another surgical fastener housed by at least one other receptacle of the plurality of receptacles.

4. The surgical clip pack of any one of the preceding claims, wherein the storage compartment (130) surrounds about a third of the annulus (112).

5. The surgical clip pack of any one of the preceding claims, wherein at least one of the plurality of receptacles (134) includes a pin (136) disposed within a proximal portion thereof, the pin configured to fit within an elbow (154) of the surgical fastener (150).

6. The surgical clip pack of any one of claims 2 to 5, further including two tabs (116), wherein each tab includes a shelf (118) at a distal portion thereof, wherein the shelf is configured to cause the tab to flex radially inwards when inserted into a slot (216) of the surgical access device (200) until the shelf exits the slot, the shelf extending over an edge of the slot thereby securing the surgical clip pack (100) to the surgical access device (200).

7. The surgical clip pack (100) of claim 1, wherein the storage compartment is an arm coupled to a side of the frame, the arm having the plurality of receptacles (134).

8. The surgical clip pack of claim 7, wherein the arm extends circumferentially about at least a portion of the frame (110).

9. The surgical clip pack of claim 7 or 8, wherein the surgical fastener (150) includes bars (156) at distal ends of arms (152) of the surgical fasteners, the bars configured to press against an internal wall (138) of the receptacle to hold the surgical fastener in the receptacle (134).

10. The surgical clip pack of any one of claims 7 to 9, wherein each receptacle (134) of the plurality of receptacles includes a pin (136) configured to stabilize the surgical fastener (150) therein, the pin being configured to fit into an elbow (154) of the surgical fastener such that the pin holds the surgical fastener in the receptacle of the plurality of receptacles.

11. The surgical clip pack of any one of the preceding claims, wherein the surgical fastener (150) is a surgical clip.

## Patentansprüche

1. Chirurgische Klammerpackung (100), umfassend:
einen ringförmigen Rahmen (110), der konfiguriert ist, um einen proximalen Abschnitt einer chirurgischen Zugangsvorrichtung (200) in Eingriff zu nehmen, wobei der Rahmen einschließt:
ein Aufbewahrungsfach (130); und
einen Ring (112), der ein Lumen (114) definiert, das einen Durchmesser, der mindestens so groß wie ein Durchmesser einer Öffnung der chirurgischen Zugangsvorrichtung ist, aufweist; und
eine Vielzahl von Aufnahmen (134), die in dem Aufbewahrungsfach angeordnet sind und umlaufend um den Rahmen herum angeordnet sind, wobei jede Aufnahme der Vielzahl von Aufnahmen konfiguriert ist, um mindestens ein chirurgisches Befestigungselement (150) aufzunehmen.

2. Chirurgische Klammerpackung nach Anspruch 1, wobei der Rahmen (110) mindestens eine Lasche (116) einschließt, die konfiguriert ist, um einen Schlitz (216), der an dem proximalen Abschnitt der chirurgischen Zugangsvorrichtung (200) angeordnet ist, in Eingriff zu nehmen.

3. Chirurgische Klammerpackung nach einem der vorstehenden Ansprüche, wobei mindestens eine Aufnahme (134) der Vielzahl von Aufnahmen konfiguriert ist, um ein chirurgisches Befestigungselement (150) einer unterschiedlichen Größe als ein anderes chirurgisches Befestigungselement, das durch mindestens eine andere Aufnahme der Vielzahl von Aufnahmen untergebracht ist, aufzunehmen.

4. Chirurgische Klammerpackung nach einem der vorstehenden Ansprüche, wobei das Aufbewahrungsfach (130) etwa ein Drittel des Rings (112) umgibt.

5. Chirurgische Klammerpackung nach einem der vorstehenden Ansprüche, wobei mindestens eine der Vielzahl von Aufnahmen (134) einen Stift (136), der in einem proximalen Abschnitt davon angeordnet ist, einschließt, wobei der Stift konfiguriert ist, um in einen Bogen (154) des chirurgischen Befestigungselements (150) zu passen.

6. Chirurgische Klammerpackung nach einem der Ansprüche 2 bis 5, die ferner zwei Laschen (116) einschließt, wobei jede Lasche einen Vorsprung (118) an einem distalen Abschnitt davon einschließt; wobei der Vorsprung konfiguriert ist, um zu bewirken, dass sich die Lasche radial nach innen biegt, wenn sie in einen Schlitz (216) der chirurgischen Zugangsvorrichtung (200) eingeführt wird, bis der Vorsprung aus dem Schlitz austritt, wobei sich der Vorsprung über eine Kante des Schlitzes erstreckt, wobei dadurch die chirurgische Klammerpackung (100) an der chirurgischen Zugangsvorrichtung (200) gesichert wird.

7. Chirurgische Klammerpackung (100) nach Anspruch 1, wobei das Aufbewahrungsfach ein Arm ist, der mit einer Seite des Rahmens gekoppelt ist, wobei der Arm die Vielzahl von Aufnahmen (134) aufweist.

8. Chirurgische Klammerpackung nach Anspruch 7, wobei sich der Arm umlaufend um mindestens einen Abschnitt des Rahmens (110) herum erstreckt.

9. Chirurgische Klammerpackung nach Anspruch 7 oder 8, wobei das chirurgische Befestigungselement (150) Stege (156) an distalen Enden von Armen (152) der chirurgischen Befestigungselemente einschließt, wobei die Stege konfiguriert sind, um gegen eine Innenwand (138) der Aufnahme zu drücken, um das chirurgische Befestigungselement in der Aufnahme (134) zu halten.

10. Chirurgische Klammerpackung nach einem der Ansprüche 7 bis 9, wobei jede Aufnahme (134) der Vielzahl von Aufnahmen einen Stift (136) einschließt, der konfiguriert ist, um das chirurgische Befestigungselement (150) darin zu stabilisieren, wobei der Stift konfiguriert ist, um in einen Bogen (154) des chirurgischen Befestigungselements zu passen, sodass der Stift das chirurgische Befestigungselement in der Aufnahme der Vielzahl von Aufnahmen hält.

11. Chirurgische Klammerpackung nach einem der vorstehenden Ansprüche, wobei das chirurgische Befestigungselement (150) eine chirurgische Klammer ist.

## Revendications

1. Boîte de clips chirurgicaux (100) comprenant :
un cadre annulaire (110) conçu pour venir en prise avec une partie proximale d'un dispositif d'accès chirurgical (200), le cadre comportant :
un compartiment de stockage (130) ; et
un anneau (112) définissant une lumière (114) qui a un diamètre au moins aussi grand qu'un diamètre d'une ouverture du dispositif d'accès chirurgical ; et
une pluralité de réceptacles (134) disposés à l'intérieur du compartiment de stockage et disposés circonférentiellement autour du cadre, chaque réceptacle de la pluralité de réceptacles étant conçu pour loger au moins une fixation chirurgicale (150).

2. Boîte de clips chirurgicaux selon la revendication 1, dans laquelle le cadre (110) comporte au moins une languette (116) conçue pour venir en prise avec une fente (216) disposée sur la partie proximale du dispositif d'accès chirurgical (200).

3. Boîte de clips chirurgicaux selon l'une quelconque revendication précédente, dans laquelle au moins un réceptacle (134) de la pluralité de réceptacles est conçu pour loger une fixation chirurgicale (150) d'une taille différente de celle d'une autre fixation chirurgicale logée par au moins un autre réceptacle de la pluralité de réceptacles.

4. Boîte de clips chirurgicaux selon l'une quelconque des revendications précédentes, dans laquelle le compartiment de stockage (130) entoure environ un tiers de l'anneau (112).

5. Boîte de clips chirurgicaux selon l'une quelconque des revendications précédentes, dans laquelle au moins l'un de la pluralité de réceptacles (134) comporte une broche (136) disposée à l'intérieur d'une partie proximale de celui-ci, la broche étant conçue pour s'insérer à l'intérieur d'un coude (154) de la fixation chirurgicale (150).

6. Boîte de clips chirurgicaux selon l'une quelconque des revendications 2 à 5, comportant en outre deux languettes (116), dans laquelle chaque languette comporte un rebord (118) au niveau d'une partie distale de celle-ci ; dans laquelle le rebord est conçu pour amener la languette à fléchir radialement vers l'intérieur lorsqu'il est inséré dans une fente (216) du dispositif d'accès chirurgical (200) jusqu'à ce que le rebord sorte de la fente, le rebord s'étendant au-dessus d'un bord de la fente, fixant ainsi la boîte de clips chirurgicaux (100) au dispositif d'accès chirurgical (200).

7. Boîte de clips chirurgicaux (100) selon la revendication 1, dans laquelle le compartiment de stockage est un bras accouplé à un côté du cadre, le bras ayant la pluralité de réceptacles (134).

8. Boîte de clips chirurgicaux selon la revendication 7, dans laquelle le bras s'étend circonférentiellement autour d'au moins une partie du cadre (110).

9. Boîte de clips chirurgicaux selon la revendication 7 ou 8, dans laquelle la fixation chirurgicale (150) comporte des barres (156) au niveau d'extrémités distales de bras (152) des fixations chirurgicales, les barres étant conçues pour s'appuyer contre une paroi interne (138) du réceptacle afin de maintenir la fixation chirurgicale dans le réceptacle (134).

10. Boîte de clips chirurgicaux selon l'une quelconque des revendications 7 à 9, dans laquelle chaque réceptacle (134) de la pluralité de réceptacles comporte une broche (136) conçue pour stabiliser la fixation chirurgicale (150) à l'intérieur de celui-ci, la broche étant conçue pour s'insérer dans un coude (154) de la fixation chirurgicale de telle sorte que la broche maintient la fixation chirurgicale dans le réceptacle de la pluralité de réceptacles.

11. Boîte de clips chirurgicaux selon l'une quelconque des revendications précédentes, dans laquelle la fixation chirurgicale (150) est un clip chirurgical.
